# EUROPÄISCHE PATENTSCHRIFT

(11) **EP 2 347 250 B1**
(45) Veröffentlichungstag und Bekanntmachung des Hinweises auf die Patenterteilung: **30.07.2014**
(21) Anmeldenummer: 09748935.5
(22) Anmeldetag: 12.10.2009
(51) Int. Cl.: G01N 27/22, G01N 33/36, G01R 27/26

(54) **VERFAHREN FÜR DEN SYMMETRIEABGLEICH EINER KAPAZITIVEN VORRICHTUNG**
METHOD FOR SYMMETRY ALIGNMENT OF A CAPACITIVE DEVICE
PROCÉDÉ DE ALIGNEMENT DE SYMETRIE D'UN DISPOSITIF CAPACITIF

(30) Priorität: 16.10.2008 CH 16462008
(43) Veröffentlichungstag der Anmeldung: 27.07.2011
(60) Teilanmeldung: 11000172.4
(73) Patentinhaber: Uster Technologies AG, 8610 Uster (CH)
(72) Erfinder: GEHRIG, Reto, 8406 Winterthur (CH)
(86) Internationale Anmeldenummer: PCT/CH2009/000329
(87) Internationale Veröffentlichungsnummer: WO 2010/043065

(56) Entgegenhaltungen:
- WO-A2-2004/083813
- DE-A1- 19 535 177
- DE-A1- 19 850 290
- GB-A- 1 061 635
- US-A- 3 684 954
- US-A- 3 757 211
- US-A- 4 706 203
- US-A- 4 772 844
- US-A1- 2001 008 478

## Beschreibung

### FACHGEBIET

Die vorliegende Erfindung liegt auf dem Gebiet der elektrischen Messvorrichtungen. Sie betrifft ein Verfahren für den Symmetrieabgleich einer Vorrichtung zur kapazitiven Untersuchung eines bewegten länglichen textilen Prüfgutes, gemäss dem Oberbegriff des ersten Patentanspruchs. Die Erfindung erlaubt ein automatisches Abgleichen der Vorrichtung.

Ein bevorzugtes Einsatzgebiet für die Erfindung ist die kapazitive Prüfung von länglichen, vorzugsweise textilen Gebilden wie Kardenband, Vorgarn, Garn oder Gewebe. Eine derartige Prüfung kann bspw. die Detektion von Fremdstoffen oder das Erkennen von Änderungen der Masse pro Längeneinheit und/oder die Messung von Feuchtigkeit im Prüfgut zum Ziel haben. Die Erfindung kann bspw. im Produktionsprozess (online) in Garnreinigern auf Spinn- oder Spulmaschinen oder in der Laborprüfung (offline) in Garnprüfgeräten eingesetzt werden.

### STAND DER TECHNIK

Es ist eine Vielzahl verschiedenartiger Vorrichtungen zur Untersuchung oder Prüfung von länglichem textilem Prüfgut wie bspw. Kardenband, Vorgarn, Garn oder Gewebe bekannt. Sie lassen sich nach ihrer Anwendung in die beiden Klassen Laborprüfung (offline) und Prüfung während des Produktionsprozesses (online) einteilen. Die Vorrichtungen verwenden verschiedene bekannte Sensorprinzipien; hier interessiert besonders das kapazitive Messprinzip. Bei diesem ist ein Messkondensator typischerweise als ebener Plattenkondensator ausgeführt und weist eine Durchgangsöffnung für das Prüfgut auf. Der Messkondensator ist Teil eines LC-Oszillators, so dass bei Anregung des LC-Oszillators eine elektrische Wechselspannung am Messkondensator anliegt. Die Durchgangsöffnung wird dadurch mit einem elektrischen Wechselfeld beaufschlagt. Das Prüfgut wird durch den Plattenkondensator hindurch bewegt und dem Wechselfeld ausgesetzt. Es wird ein elektrisches Ausgangssignal des Plattenkondensators detektiert. In einer Auswerteschaltung werden aus dem Ausgangssignal dielektrische Eigenschaften des Prüfgutes ermittelt. Aus den dielektrischen Eigenschaften werden Parameter des Prüfgutes wie Masse pro Längeneinheit und/oder Materialzusammensetzung bestimmt. Ein kapazitiver Garn- oder Bandsensor ist z. B. in der GB-638,365 A beschrieben.

Um genaue, von äusseren Einflüssen wie Lufttemperatur oder Luftfeuchtigkeit nicht beeinflusste Messungen durchführen zu können, wird häufig eine Kompensationsmethode angewendet. Zu diesem Zweck beinhaltet die Vorrichtung nebst dem eigentlichen Messkondensator einen Referenzkondensator. Dieser kann durch Zufügen einer dritten, parallel zu den beiden Messkondensatorplatten angeordneten Kondensatorplatte gebildet werden, wobei die drei Kondensatorplatten zu einer kapazitiven Messschaltung zusammen geschaltet werden. Beispiele für Messschaltungen und geeignete Auswerteschaltungen für deren Ausgangssignale finden sich in den Schriften EP-0'924'513 A1, WO-2006/105676 A1 und WO-2007/115416 A1.

Ohne Prüfgut sollte die Messschaltung bei angelegter Wechselspannung ein Ausgangssignal mit dem Wert Null liefern. In der Praxis genügt es jedoch wegen verschiedener Unvollkommenheiten realer elektrischer Komponenten nicht, die Messschaltung symmetrisch aufzubauen, um ohne Prüfgut ein Nullsignal zu erhalten. Jede einzelne Messschaltung muss zur Symmetrisierung individuell abgeglichen werden. Der Symmetrieabgleich erfolgt in der Produktion durch den Hersteller und bei Bedarf während des Unterhalts durch eine Serviceperson. Zu diesem Zweck wird üblicherweise die Kapazität mindestens eines zur Messkapazität parallel geschalteten Trimmkondensators verändert. Das Trimmen erfolgt manuell mit einem geeigneten Werkzeug, z. B. einem Schraubendreher. Alternativ wird das bekannte Verfahren des Lasertrimmens angewendet. In jedem Fall muss die Vorrichtung für den Abgleich geöffnet werden. Ein solcher manueller Abgleich ist mühsam, zeitaufwändig und kostspielig.

Die US-3,684,953 A befasst sich mit einer Vorrichtung und einem Verfahren zur quantitativen Bestimmung einer Eigenschaft eines dielektrischen Prüfgutes, insbesondere seines relativen Feuchtegehaltes. Das Prüfgut wird in einen Messkondensator eingebracht. An den Messkondensator wird ein elektrisches Wechselsignal angelegt, das von einem Oszillator mit einem nachgeschalteten Verstärker erzeugt wird. Ein am Messkondensator abgegriffenes Signal wird demoduliert und mit dem am Messkondensator angelegten, ebenfalls demodulierten Wechselsignal verglichen, indem bspw. ein Quotient der beiden Signale gebildet wird. Aus dem Vergleich wird auf den Feuchtegehalt des Prüfgutes geschlossen. Um den Dynamikbereich der Demodulatoren einzuhalten, wird die Verstärkung des Verstärkers mit dem demodulierten Ausgangssignal des Messkondensators geregelt. Für den Nullabgleich ohne Prüfgut ist ein parallel zum Messkondensator geschalteter, veränderlicher Abgleichkondensator vorgesehen.

Aus der US-4,843,879 A ist ein Gerät für die kapazitive Qualitätskontrolle von textilen Fäden bekannt. Es beinhaltet eine Doppelkondensatoranordnung mit einem Messkondensator und einem Referenzkondensator. Die Doppelkondensatoranordnung ist in einem elektrischen Schaltkreis eingebaut. Der Schaltkreis beinhaltet einen Oszillator zum Anlegen zweier Wechselspannungen mit entgegengesetzten Phasen an die beiden äusseren Kondensatorelektroden der Doppelkondensatoranordnung. In jedem Ast zwischen dem Oszillator und der jeweiligen Elektrode befinden sich ein Signalverstärker und ein Abgleichkondensator. Die Abgleichkondensatoren dienen dem Abgleich des Ausgangssignals der Doppelkondensatoranordnung ohne Prüfgut auf den Wert Null.

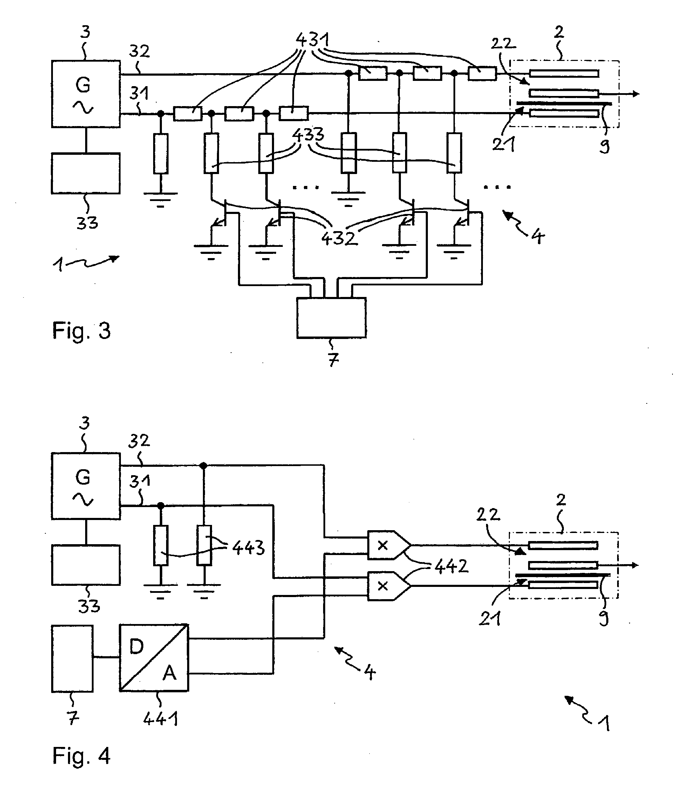

Die DE- 195'35'177 A1 offenbart ein Verfahren und eine Vorrichtung zum Prüfen der Struktur on Garnen. In der Ausführungsform gemäss Figur 4 jener Schrift wird ein Garn durch einen Messkondensator geführt. Der Messkondensator befindet sich in einer Kapazitätsmessbrücke, in dem auch eine variable Kapazität eingebaut ist. Die variable Kapazität wird in einem geschlossenen Regelkreis dem Messkondensator so nachgestellt, dass sich in einem Abgleichmessgerät der Kapazitätsmessbrücke stets der Wert Null ergibt. Die Einstellwerte für die variable Kapazität sind ein Mass für die im Messkondensator befindliche Garnmasse.

### DARSTELLUNG DER ERFINDUNG

Es ist deshalb eine Aufgabe der vorliegenden Erfindung, ein Verfahren für den Symmetrieabgleich einer Vorrichtung zur kapazitiven Untersuchung eines bewegten länglichen textilen Prüfgutes anzugeben, welches die obigen Nachteile nicht aufweist. Die Vorrichtung soll einfach, schnell, kostengünstig und insbesondere automatisch abgleichbar sein.

Diese und andere Aufgaben werden durch das erfindungsgemässe Verfahren, wie es in Anspruch 1 definiert ist, gelöst. Vorteilhafte Ausführungsformen sind in den abhängigen Patentansprüchen angegeben.

Die Erfindung beruht auf der Idee, Abgleichmittel in einem elektrischen Pfad zwischen einem Wechselsignalgenerator und einer die Kondensatoranordnung beinhaltenden Messschaltung vorzusehen, die mittels eines elektrischen Steuersignals steuerbar sind, um ein Ausgangssignal der Vorrichtung abzugleichen. Mit solchen externen Abgleichmitteln wird die Vorrichtung abgeglichen, ohne in die Kondensatoranordnung selbst einzugreifen. Dies eröffnet die Möglichkeit eines automatischen Abgleichs der Vorrichtung.

Unter dem Begriff "Kondensatoranordnung" wird in dieser Schrift eine Anordnung mit zwei Körpern, die vom elektrischen Wechselsignal des Wechselsignalgenerators ungleichartig aufladbar und durch mindestens ein Dielektrikum voneinander getrennt sind, verstanden. In einer bevorzugten Ausführungsform handelt es sich bei der Kondensatoranordnung um einen Kondensator mit zwei voneinander beabstandeten Platten, zwischen denen sich Luft befindet und zwischen die ein zu untersuchendes bewegtes längliches textiles Prüfgut einführbar ist. Unter dem Begriff "elektrisches Wechselsignal" wird in dieser Schrift ein elektrisches Spannungs- oder Stromsignal mit mindestens einem sich zeitlich ändernden, vorzugsweise periodischen Anteil (AC-Anteil) verstanden, dem zusätzlich ein zeitlich im Wesentlichen konstanter Anteil (DC-Anteil, Offset) überlagert sein kann.

Die Vorrichtung zur Bestimmung mindestens einer dielektrischen Eigenschaft einer Kondensatoranordnung beinhaltet mindestens einen Wechselsignalgenerator zum Anlegen eines elektrischen Wechselsignals an die Kondensatoranordnung. Sie beinhaltet ferner eine Auswerteschaltung zur Auswertung mindestens einer elektrischen Messgrösse eines an der Messschaltung abgegriffenen elektrischen Signals. Ausserdem beinhaltet die Vorrichtung Abgleichmittel, die in einem elektrischen Pfad zwischen dem mindestens einen Wechselsignalgenerator und der Messschaltung angeordnet sind und mittels derer mindestens ein Parameter des elektrischen Wechselsignals derart veränderbar ist, dass ein Ausgangssignal der Auswerteschaltung bei definierten, konstanten Bedingungen einen bestimmten Wert, vorzugsweise Null, annimmt. Es sind Steuermittel zur Abgabe eines elektrischen Steuersignals an die Abgleichmittel vorhanden, mittels dessen die Veränderung des mindestens einen Parameters steuerbar ist.

In einer bevorzugten Ausführungsform weist die Vorrichtung eine Rückkopplung auf, mittels deren ein Ausgangssignal der Messschaltung oder der Auswerteschaltung auf die Steuermittel einwirkt. Die Kondensatoranordnung ist vorzugsweise vom Wechselsignalgenerator derart abgekoppelt, dass sie die Grundfrequenz und die Signalform des angelegten Wechselsignals nicht wesentlich beeinflusst.

Zur Realisierung der Abgleichmittel werden verschiedene beispielhafte Varianten vorgeschlagen, wobei die Aufzählung nicht abschliessend ist:
- Die Abgleichmittel beinhalten eine Mehrzahl von elektrischen Widerständen, die einzeln oder gruppenweise zu- oder wegschaltbar sind.
- Die Abgleichmittel beinhalten einen Modulator für eine Amplitudenmodulation des elektrischen Wechselsignals.
- Die Abgleichmittel beinhalten einen Verstärker mit variabler oder programmierbarer Verstärkung zur Verstärkung des elektrischen Wechselsignals.
- Die Abgleichmittel beinhalten ein digitales Potenziometer oder einen Rejustor.
- Die Abgleichmittel beinhalten eine Kapazitätsdiode.

Die Messschaltung beinhaltet einen Referenzkondensator, welcher in Serie zur Kondensatoranordnung geschaltet ist. Dabei kann der mindestens eine Wechselsignalgenerator dazu eingerichtet sein, an die Kondensatoranordnung bzw. an den Referenzkondensator zwei elektrische Wechselspannungen mit entgegengesetzten Phasen anzulegen. Die Abgleichmittel können im elektrischen Pfad zwischen dem Wechselsignalgenerator und der Kondensatoranordnung und/oder im elektrischen Pfad zwischen dem mindestens einen Wechselsignalgenerator und dem Referenzkondensator angeordnet sein.

Die Vorrichtung wird zur kapazitiven Untersuchung eines bewegten länglichen textilen Prüfgutes wie Kardenband, Vorgarn, Garn oder Gewebe verwendet, wobei das bewegte Prüfgut die Kondensatoranordnung beeinflusst.

Das erfindungsgemässe Verfahren dient dem Symmetrieabgleich einer Vorrichtung zur kapazitiven Untersuchung eines bewegten länglichen textilen Prüfgutes wie Kardenband, Vorgarn, Garn oder Gewebe mittels einer Kondensatoranordnung. Die Vorrichtung beinhaltet mindestens einen Wechselsignalgenerator zum Anlegen eines elektrischen Wechselsignals an die Kondensatoranordnung, eine Auswerteschaltung zur Auswertung mindestens einer elektrischen Messgrösse eines an der Messschaltung abgegriffenen elektrischen Signals, Abgleichmittel, die in einem elektrischen Pfad zwischen dem mindestens einen Wechselsignalgenerator und der die Kondensatoranordnung beinhaltenden Messschaltung angeordnet sind und mittels derer mindestens ein Parameter des elektrischen Wechselsignals derart veränderbar ist, dass ein Ausgangssignal der Auswerteschaltung bei definierten, konstanten Bedingungen einen bestimmten Wert, vorzugsweise Null, annimmt, und Steuermittel zur Abgabe eines elektrischen Steuersignals an die Abgleichmittel, mittels dessen die Veränderung des mindestens einen Parameters steuerbar ist. Die Kondensatoranordnung wir ohne Prüfgut im Wesentlichen zeitlich unverändert belassen, ein elektrisches Wechselsignal wird von dem mindestens einen Wechselsignalgenerator erzeugt und an die Kondensatoranordnung angelegt, ein elektrisches Ausgangssignal wird der Messschaltung abgegriffen und mindestens eine elektrische Messgrösse des an der Messschaltung abgegriffenen elektrischen Ausgangssignals wird durch die Auswerteschaltung ausgewertet. Mindestens ein Parameter des elektrischen Wechselsignals wird in dem elektrischen Pfad zwischen dem mindestens einen Wechselsignalgenerator und der Messschaltung derart durch die Abgleichmittel verändert, dass ein Ausgangssignal der Auswertung bei definierten, konstanten Bedingungen einen bestimmten Wert, vorzugsweise Null, annimmt. Die Veränderung des mindestens einen Parameters mit dem elektrischen Steuersignal wird durch die Steuermittel gesteuert, und das elektrische Steuersignal wird durch das Ausgangssignal beeinflusst.

Als unmittelbare Vorteile, welche die Erfindung bietet, können unter anderen folgende genannt werden:
- Erstens braucht der Abgleich nicht in der Messschaltung zu erfolgen. Die Messschaltung ist der wohl empfindlichste Teil der kapazitiven Vorrichtung und ist somit äusserst empfindlich auf Störungen und Nichtlinearitäten. Indem sie Abgleichmittel ausserhalb der Messschaltung anordnet, vermeidet die Erfindung derartige Störungen in der Messschaltung.
- Zweitens kann erfindungsgemäss der Abgleich mit einer beliebigen Genauigkeit erfolgen. Es kann beispielsweise ein digitales Potenziometer eingesetzt werden, dessen Widerstand über eine Schnittstelle mit einer beliebig grossen Auflösung gesteuert werden kann.
- Drittens kann mit der Erfindung das Signal/Rauschverhältnis für all jene kapazitiven Sensoren merklich verbessert werden, bei welchen der Abgleich bisher durch einen trimmbaren Kondensator in der Messschaltung erfolgte. Dies deshalb, weil ein trimmbares Element in der Messschaltung dauernd eine Wirkung auf die Messschaltung ausübt.

Die Erfindung ist nützlich zum Abgleichen der Vorrichtung. Dank der Erfindung wird der Abgleich der Vorrichtung einfach, schnell und kostengünstig. Das Gerät muss nicht geöffnet werden, um die Messschaltung abzugleichen. Der Abgleich kann jederzeit erfolgen. Er kann automatisch vom Gerät selbst vorgenommen werden, ohne Eingriff einer Bedienungsperson. Dadurch wird es auch möglich, einen Abgleich zu irgendeinem beliebigen Zeitpunkt durchzuführen. So könnte ein Abgleich vor jeder Messung, vor jeder zehnten Messung, bei wesentlichen Änderungen von Langzeiteigenschaften des Ausgangssignals oder nach einer grösseren Änderung der Umgebungsbedingungen automatisch durchgeführt werden. Dies kann eine Erhöhung der Messgenauigkeit, der Messzuverlässigkeit, der Reproduzierbarkeit und allgemein eine Verbesserung der Messresultate zur Folge haben.

### AUFZÄHLUNG DER ZEICHNUNGEN

Nachfolgend wird die Erfindung anhand der schematischen Zeichnungen detailliert erläutert.
- Figur 1: zeigt ein Blockdiagramm der Vorrichtung.
- Figuren 2-9: zeigen Schaltschemata von verschiedenen Ausführungsformen der Vorrichtung.

### AUSFÜHRUNG DER ERFINDUNG

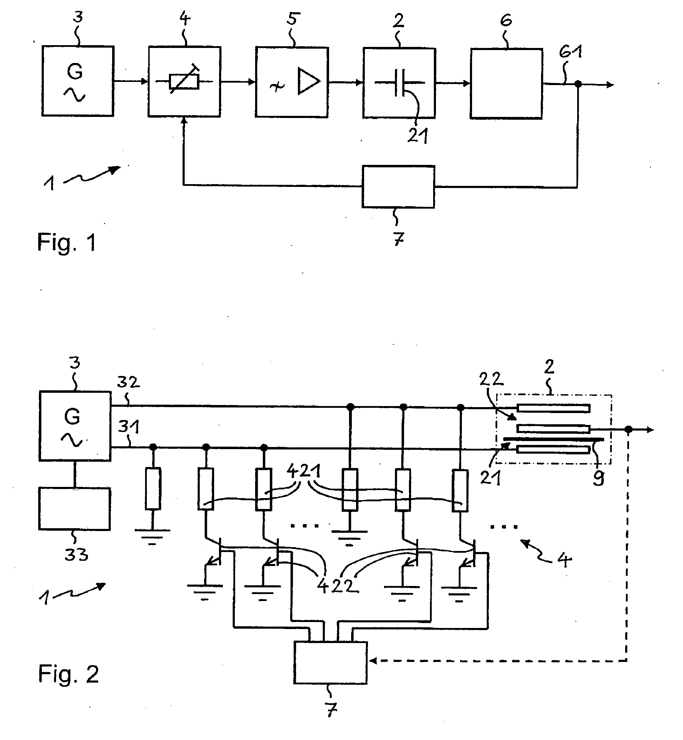

Figur 1 erklärt anhand eines einfachen Blockdiagramms die Erfindung. Die Vorrichtung 1 dient zum Ausmessen einer Kapazität 21, welche in einer Messschaltung 2 eingesetzt sein kann. An die Messschaltung 2 wird ein elektrisches Wechselsignal, z. B. eine Wechselspannung, angelegt. Zur Erzeugung des Wechselsignals ist ein Wechselsignalgenerator 3 vorgesehen. Das vom Wechselsignalgenerator 3 erzeugte Wechselsignal kann noch in einer Filter- und/oder Verstärkerstufe 5 gefiltert und/oder verstärkt werden. Die Filter- und/oder Verstärkerstufe 5 dient vorzugsweise auch dazu, die auszumessende Kapazität 21 vom Wechselsignalgenerator 3 derart abzukoppeln, dass sie Parameter des vom Wechselsignalgenerator 3 erzeugten Wechselsignals, z. B. Frequenz, Phase und/oder Amplitude des Wechselsignals, nicht beeinflusst. Der Einfachheit halber ist die Filter- und/oder Verstärkerstufe 5 in den nachfolgenden Figuren nicht mehr eingezeichnet. Der Messschaltung 2 ist eine Auswerteschaltung 6 nachgeschaltet, die ein Ausgangssignal der Messschaltung 2 auswertet. Die Auswerteschaltung 6 kann als Rechner ausgebildet sein. Sie gibt ihrerseits auf einer Ausgangsleitung 61 ein Ausgangssignal aus, das ein Mass für die auszumessende Kapazität 21 ist. In der Vorrichtung 1 können selbstverständlich noch weitere Stufen vorgesehen sein. Insbesondere kann es vorteilhaft oder nötig sein, zwischen der Messschaltung 2 und der Auswerteschaltung 6 Filter, Verstärker und/oder Mischer einzubauen, die jedoch in den beiliegenden Zeichnungen der Einfachheit halber nicht dargestellt sind.

Erfindungsgemäss sind in einem elektrischen Pfad zwischen dem Wechselsignalgenerator 3 und der Messschaltung 2 Abgleichmittel 4 zum Abgleichen der Vorrichtung 1 angeordnet, mittels derer mindestens ein Parameter, z. B. die Amplitude, des elektrischen Wechselsignals veränderbar ist. Während des Abgleichs der Vorrichtung 1 sollte darauf geachtet werden, dass sich die auszumessende Kapazität 21 und allenfalls andere in der Messschaltung 2 vorhandene Kapazitäten zeitlich nicht ändern. Es sollten also zeitlich konstante Umgebungsbedingungen wie Temperatur und Luftfeuchtigkeit herrschen, oder es sollten entsprechende Änderungen kompensiert werden. Da die auszumessende Kapazität 21 als Kondensator zur Aufnahme von Prüfgut ausgebildet ist (wie in den nachfolgenden Figuren dargestellt), sollte sich während des Abgleichs kein Prüfgut im Kondensator befinden. Zum Abgleich der Vorrichtung 1 wird ein elektrisches Wechselspannungssignal an die auszumessende Kapazität 21 angelegt. Die Abgleichmittel 4 verändern mindestens einen Parameter des elektrischen Wechselsignals derart, dass das Ausgangssignal der Vorrichtung 1 auf der Ausgangsleitung 61 einen bestimmten Wert annimmt, vorzugsweise null wird. Zu diesem Zweck können die Abgleichmittel 4 manuell von einer Person eingestellt werden, bspw. nach der Herstellung oder beim Unterhalt der Vorrichtung 1. Alternativ können die Abgleichmittel 4 automatisch eingestellt werden. Die automatische Einstellung kann in einer speziell dafür vorgesehenen Steuereinheit 7 oder in der Auswerteeinheit 6 erfolgen. In Figur 1 ist eine Rückkopplung von der Ausgangsleitung 61 zu den Abgleichmitteln 4 eingezeichnet, die ein vom Ausgangssignal der Auswerteeinheit 6 bzw. der Messschaltung 2 abhängiges Steuersignal an die Abgleichmittel 4 abgibt. Mit einer automatischen Einstellung der Abgleichmittel 4 kann die Vorrichtung 1 automatisch abgeglichen werden. Somit liegt ein geschlossener Regelkreis vor, in dem das Ausgangssignal die Regelgrösse ist, die auf den Sollwert Null geregelt werden soll, die Steuereinheit 7 als Regler wirkt und das Steuersignal der Stellwert ist. Alternativ kann die Regelgrösse vor statt hinter der Auswerteeinheit 6 abgegriffen werden.

Eine erste Ausführungsform der Vorrichtung 1 ist in **Figur 2** dargestellt. Die Vorrichtung 1 dient zur kapazitiven Ausmessung eines Prüfgutes 9, z. B. eines Garns. Dazu wird das Prüfgut 9 in einen Messkondensator 21, z. B. einen ebenen Plattenkondensator, eingebracht. Der Messkondensator 21 bildet zusammen mit einem in Serie geschalteten Referenzkondensator 22 und möglicherweise weiteren (nicht eingezeichneten) Komponenten eine kapazitive Messschaltung 2. Der Messschaltung 2 kann, wie in Figur 1, eine Auswerteeinheit 6 nachgeschaltet sein, die jedoch in Figur 2 und in den nachfolgenden Figuren der Übersichtlichkeit halber nicht eingezeichnet ist.

Der Wechselsignalgenerator 3 kann z. B. ein Synthesizer, vorzugsweise ein direkter digitaler Synthesizer (direct digital synthesizer, DDS) sein. Der Synthesizer 3 kann von einer digitalen Schnittstelle 33 angesteuert werden. Der Synthesizer 3 hat vorzugsweise zwei Ausgänge 31, 32 für zwei elektrische Wechselsignale, die im Wesentlichen identisch, aber um 180° gegeneinander phasenverschoben sind. Dem Fachmann sind auch andere Wechselsignalgeneratoren bekannt, die zum Anlegen eines elektrischen Wechselsignals an die Messschaltung 2 geeignet sind, z. B. aus der folgenden Menge: RC-Oszillator, LC-Oszillator, Quarz-Oszillator, Oszillator mit Keramikresonator, Oszillator mit SAW-Komponente (akustische Oberflächenwellen, surface acoustic waves, SAW), Oszillator mit Logikbausteinen, Phasenregelschleife (phase-locked loop, PLL), Pulsweitenmodulator (pulse-width modulator, PWM), Kippstufe.

Im Ausführungsbeispiel von Figur 2 beinhalten die Abgleichmittel 4 eine Mehrzahl von parallel zueinander geschalteten Widerständen 421, die einzeln oder gruppenweise durch Schalter 422, z. B. Hochfrequenztransistoren, zu- und wegschaltbar sind. Dadurch ergibt sich ein veränderbarer Gesamtwiderstand, über welchem eine Spannung entsprechend abfällt, die zudem vom durch den Synthesizer abgegebenen Strom abhängt. Die Widerstände 421 können nur an einem der beiden Synthesizerausgänge oder, wie im Ausführungsbeispiel von Figur 1, an beiden Synthesizerausgängen 31, 32 angebracht sein. Die Schalter 422 können durch eine entsprechende digitale Schnittstelle 7 gesteuert werden.

Zum automatischen Abgleichen der Vorrichtung 1 kann eine Rückkopplung zwischen dem Ausgangssignal der Messschaltung 2 und der digitalen Schnittstelle 7, welche die Schalter 422 ansteuert, vorgesehen sein. Um die Vorrichtung abzugleichen, wird in einer bevorzugten Anwendung kein Prüfgut 9 in den Messkondensator eingerührt, und es wird auf zeitlich möglichst unveränderliche Umgebungsbedingungen wie Luftfeuchtigkeit und Temperatur geachtet. Mittels des Synthesizers 3 wird ein elektrisches Wechselsignal an die Messschaltung 2 angelegt, und die Stellung der Schalter 422 wird durch die digitale Schnittstelle 7 so geregelt, dass das Ausgangssignal der Messschaltung 2 null ist. Wenn dies der Fall ist, dann ist die Vorrichtung 1 abgeglichen, und die Stellung der Schalter 422 wird gespeichert und für die eigentlichen Messungen mit dem Prüfgut 9 beibehalten. Die digitale Schnittstelle 7 entspricht somit der Steuereinheit 7 von Figur 1 und wirkt als Regler in einem geschlossenen Regelkreis.

Die Möglichkeit der Rückkopplung und Regelung besteht in allen Ausführungsformen der Vorrichtung 1, ist aber der Einfachheit halber in den nachfolgenden Figuren nicht mehr eingezeichnet. Aus demselben Grund ist auch in den Figuren 2-9 die fakultative Filter- und/oder Verstärkerstufe 5 (siehe Figur 1) nicht eingezeichnet.

Figur 3 zeigt eine zweite Ausführungsform der Vorrichtung 1. Hier sind die Abgleichmittel 4 als Widerstandsleitern ausgebildet. Eine solche Widerstandsleiter beinhaltet im Wesentlichen eine Mehrzahl von in Serie geschalteten Widerständen 431, die einzeln oder Gruppenweise durch Schalter 432 über weitere Widerstände 433 zu- oder weggeschaltet werden können. Durch geeignete Schalterstellungen, die wiederum von einer digitalen Schnittstelle 7 gesteuert werden können, kann ein Abgleich der Vorrichtung 1 erreicht werden.

In einer dritten Ausführungsform der Vorrichtung 1 gemäss Figur 4 kommt ein differenzieller Digital/Analog-Wandler 441 mit zwei Ausgängen beliebiger Bit-Breite zum Einsatz. Der Digital/Analog-Wandler 441 wird von einer geeigneten digitalen Schnittstelle 7 gesteuert. Er erzeugt eine Spannung, die proportional zur Verstimmung bzw. zum Abgleich der Messschaltung 2 ist. Mit dieser Spannung wird in einem analogen Mischer oder Multiplizierer 442 die Wechselspannung des Synthesizers 3 moduliert, und das so modulierte Signal wird an die Messschaltung 2 angelegt. So kann die Amplitude der vom Synthesizer 3 erzeugten Wechselspannung beliebig eingestellt werden, bis die Vorrichtung 1 abgeglichen ist. Die beiden in Figur 4 eingezeichneten Widerstände 443 dienen der Wandlung eines vom Synthesizer 3 gelieferten Wechselstroms in eine Wechselspannung. Sie entfallen, falls der Synthesizer 3 bereits als Wechselspannungsquelle ausgebildet ist. Statt des differenziellen Digital/Analog-Wandlers 441 könnten zwei einpolige Digital/Analog-Wandler eingesetzt werden.

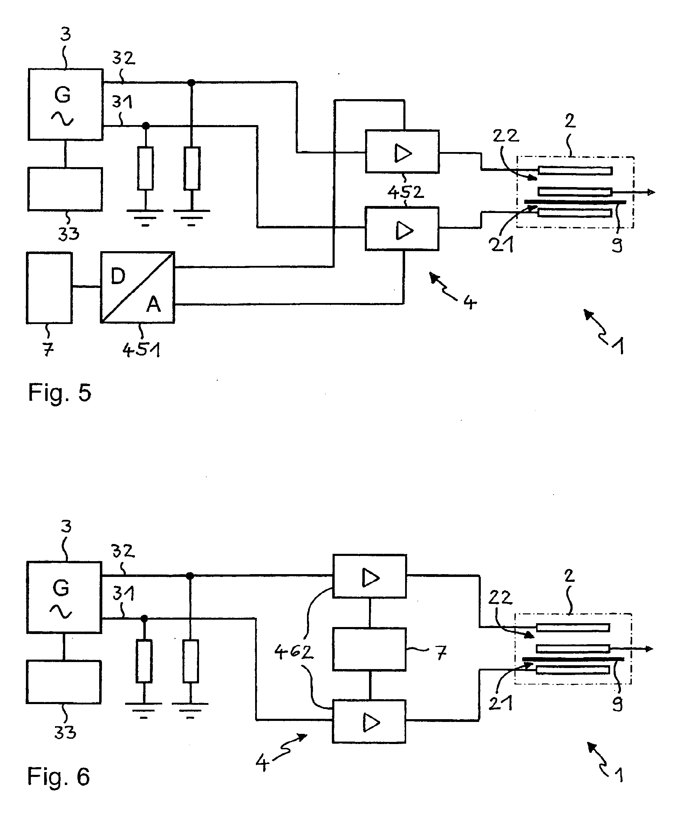

Eine vierte Ausführungsform der Vorrichtung 1, wie sie in **Figur 5** dargestellt ist, funktioniert nach dem Prinzip des spannungsgesteuerten Verstärkers. Sie beinhaltet zwei spannungsgesteuerte variable Verstärker 452 (variable gain amplifier, VGA) für die beiden Ausgangssignale des Synthesizers 3. Die Verstärkungen der variablen Verstärker 452 werden z. B. von einem Digital/Analog-Wandler 451 gesteuert, der seinerseits von einer digitalen Schnittstelle 7 gesteuert wird. Durch Einstellen von geeigneten Verstärkungen für die beiden Ausgangssignale des Synthesizers 3 kann ein Abgleich der Vorrichtung 1 erzielt werden.

Analog zur Ausführungsform von Figur 5 funktioniert eine fünfte Ausführungsform der Vorrichtung 1, die in **Figur 6** gezeigt ist. Statt der analog gesteuerten variablen Verstärker 452 kommen hier jedoch Verstärker 462 mit programmierbarer Verstärkung (programmable gain amplifier, PGA) zum Einsatz. Sie werden von einer geeigneten digitalen Schnittstelle 7 angesteuert. Der Digital/Analog-Wandler 451 entfällt bei dieser Ausführungsform.

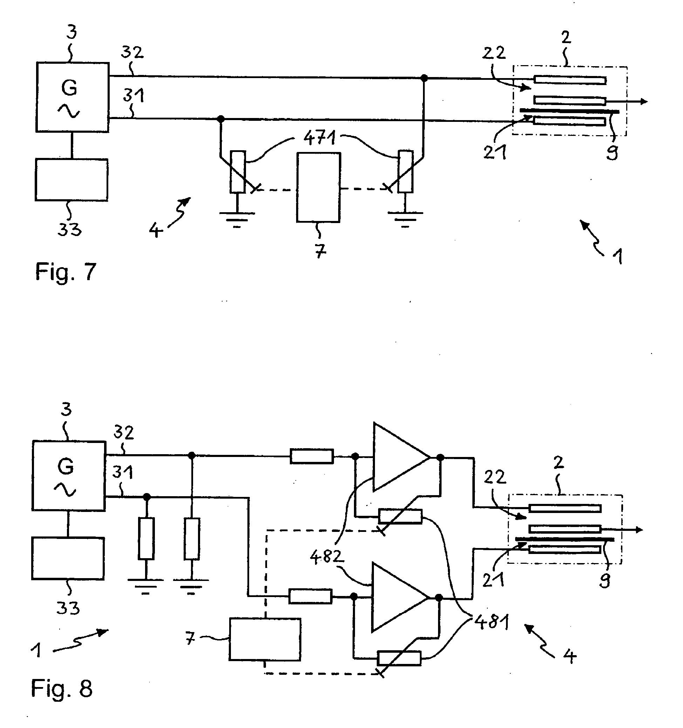

Eine sechste Ausführungsform der Vorrichtung 1 gemäss **Figur 7** verwendet digitale Potenziometer oder Rejustoren 471. Diese können über eine oder zwei digitale Schnittstellen 7 angesteuert werden, wodurch ihr Widerstand so verändert wird, dass ein gezielter Spannungsabfall durch den vom Synthesizer 3 erzeugten Wechselstrom entsteht. Falls der Synthesizer 3 als Wechselspannungsquelle ausgebildet ist, kann die Vorrichtung 1 alternativ als Spannungsteiler beschaltet sein.

Auch in einer siebten Ausführungsform der Vorrichtung 1, die in **Figur 8** dargestellt ist, werden digitale Potenziometer oder Rejustoren 481 eingesetzt, und zwar jeweils bspw. in der Gegenkopplung eines Verstärkers 482, welcher zwischen dem Synthesizer 3 und der Messschaltung 2 ein vom Synthesizer 3 erzeugtes Wechselsignal verstärkt.

Statt der digitalen Potenziometer 481 könnten in den Ausführungsformen der Figuren 7 und 8 alternativ herkömmliche, analoge Potenziometer eingesetzt werden. Mit ihnen könnte eine Bedienungsperson die Vorrichtung 1 manuell abgleichen.

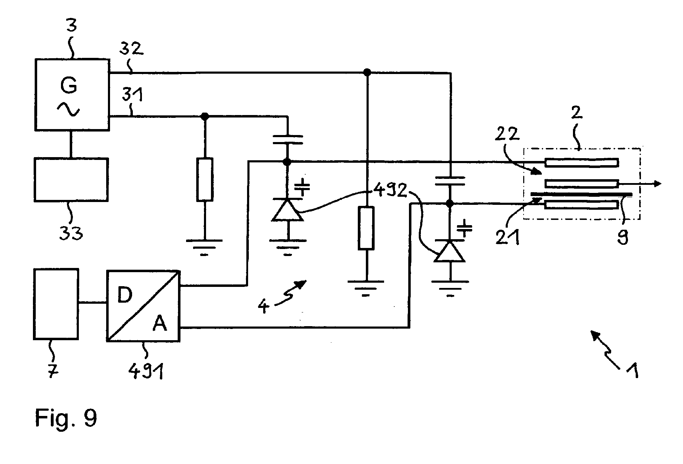

In einer achten Ausführungsform der Vorrichtung 1 gemäss **Figur 9** werden mit Kapazitätsdioden 492 kapazitive Spannungsteiler gebildet. Die Spannungen für die Steuerung der Kapazitätsdioden 492 werden z. B. von einem differenziellen Digital/Analog-Wandler 491 mit zwei Ausgängen geliefert, der seinerseits von einer digitalen Schnittstelle 7 gesteuert wird. Alternativ zum differenziellen Digital/AnalogWandler 491 könnten wiederum zwei einpolige Digital/Analog-Wandler eingesetzt werden.

Selbstverständlich ist die vorliegende Erfindung nicht auf die oben diskutierten Ausführungsformen beschränkt. Bei Kenntnis der Erfindung wird der Fachmann weitere Varianten herleiten können, die auch zum Gegenstand der vorliegenden Erfindung gehören. Solche Varianten können z. B. Kombinationen der oben diskutierten Ausführungsformen sein. Dem Fachmann sind an sich viele elektrische Komponenten bekannt, die als Abgleichmittel 4 für die Vorrichtung 1 in Frage kommen.

### BEZUGSZEICHENLISTE

- 1: Vorrichtung

- 2: Messschaltung
- 21: auszumessende Kapazität, Messkondensator
- 22: Referenzkondensator

- 3: Wechselsignalgenerator
- 31, 32: Ausgänge des Wechselsignalgenerators
- 33: digitale Schnittstelle zur Ansteuerung des Wechselsignalgenerators

- 4: Abgleichmittel
- 411: Digital/Analog-Wandler
- 421: Widerstände
- 422: Schalter
- 431, 433: Widerstände
- 432: Schalter
- 441: Digital/Analog-Wandler
- 442: Multiplizierer
- 451: Digital/Analog-Wandler
- 452: variabler Verstärker
- 462: Verstärker mit variabler Verstärkung
- 471: digitaler Potenziometer oder Rejustor
- 481: digitaler Potenziometer oder Rejustor
- 482: Verstärker
- 491: Digital/Analog-Wandler
- 492: Kapazitätsdiode

- 5: Filter- und/oder Verstärkerstufe

- 6: Auswerteschaltung

- 7: Steuereinheit

- 9: Prüfgut

## Patentansprüche

1. Verfahren für den Symmetrieabgleich einer Vorrichtung (1) zur kapazitiven Untersuchung eines bewegten länglichen textilen Prüfgutes (9) wie Kardenband, Vorgarn, Garn oder Gewebe mittels einer Kondensatoranordnung (21), welche Vorrichtung (1)
mindestens einen Wechselsignalgenerator (3) zum Anlegen eines elektrischen Wechselsignals an die Kondensatoranordnung (21),
eine Auswerteschaltung (6) zur Auswertung mindestens einer elektrischen Messgrösse eines an einer die Kondensatoranordnung (21) beinhaltenden Messschaltung (2) abgegriffenen elektrischen Signals, Abgleichmitteln (4), die in einem elektrischen Pfad zwischen dem mindestens
einen Wechselsignalgenerator (3) und der Messschaltung (2) angeordnet sind und mittels derer mindestens ein Parameter des elektrischen Wechselsignals derart veränderbar ist, dass ein Ausgangssignal der Auswerteschaltung (6) bei definierten, konstanten Bedingungen einen bestimmten Wert, vorzugsweise Null, annimmt, und Steuermittel (7) zur Abgabe eines elektrischen Steuersignals an die Abgleichmittel (4), mittels dessen die Veränderung des mindestens einen Parameters steuerbar ist,
beinhaltet,
wobei
die Kondensatoranordnung (21) ohne Prüfgut (9) im Wesentlichen zeitlich unverändert belassen wird,
ein elektrisches Wechselsignal von dem mindestens einen Wechselsignalgenerator (3) erzeugt und an die Kondensatoranordnung (21) angelegt wird,
ein elektrisches Ausgangssignal der Messschaltung (2) abgegriffen wird, mindestens eine elektrische Messgrösse des an der Messschaltung (2) abgegriffenen elektrischen Ausgangssignals durch die Auswerteschaltung (6)
ausgewertet wird,
mindestens ein Parameter des elektrischen Wechselsignals in dem elektrischen Pfad zwischen dem mindestens einen Wechselsignalgenerator (3) und der Messschaltung (2) derart durch die Abgleichmittel (4) verändert wird, dass ein Ausgangssignal der Auswertung bei definierten, konstanten Bedingungen einen bestimmten Wert, vorzugsweise Null, annimmt,
die Veränderung des mindestens einen Parameters mit dem elektrischen Steuersignal durch die Steuermittel (7) gesteuert wird, und
das elektrische Steuersignal durch das Ausgangssignal beeinflusst wird.

2. Verfahren nach Anspruch 1, wobei die Beeinflussung des elektrischen Steuersignals durch das Ausgangssignal automatisch in einem geschlossenen Regelkreis erfolgt.

3. Verfahren nach einem der vorangehenden Ansprüche, wobei die Vorrichtung (1) eine Rückkopplung aufweist, mittels deren ein Ausgangssignal der Messschaltung (2) oder der Auswerteschaltung (6) auf die Steuermittel (7) einwirkt.

4. Verfahren nach einem der vorangehenden Ansprüche, wobei die Abgleichmittel (4) eine Mehrzahl von elektrischen Widerständen (421, 431, 433) beinhalten, die einzeln oder gruppenweise zu- oder wegschaltbar sind.

5. Verfahren nach einem der vorangehenden Ansprüche, wobei die Abgleichmittel (4) einen Modulator (442) für eine Amplitudenmodulation des elektrischen Wechselsignals beinhalten.

6. Verfahren nach einem der vorangehenden Ansprüche, wobei die Abgleichmittel (4) einen Verstärker (452, 462) mit variabler oder programmierbarer Verstärkung zur Verstärkung des elektrischen Wechselsignals beinhalten.

7. Verfahren nach einem der vorangehenden Ansprüche, wobei die Abgleichmittel (4) ein digitales Potenziometer oder einen Rejustor (471, 481) beinhalten.

8. Verfahren nach einem der vorangehenden Ansprüche, wobei die Abgleichmittel (4) eine Kapazitätsdiode (492) beinhalten.

## Claims

1. A method for the symmetric balancing of an apparatus (1) for the capacitive examination of a moved elongated textile test material (9) such as card sliver, roving, yarn or fabric by means of a capacitor arrangement (21), which apparatus (1) contains
at least one alternating signal generator (3) for applying an electric alternating signal to the capacitor arrangement (21),
an evaluation circuit (6) for evaluating at least one electric measuring quantity of an electric signal tapped from a measuring circuit (2) comprising the capacitor arrangement (21),
balancing means (4) which are arranged in an electric path between the at least one alternating signal generator (3) and the measuring circuit (2) and by means of which at least one parameter of the electric alternating signal is changeable in such a way that an output signal of the evaluation circuit (6) assumes a specific value, preferably zero, under defined constant conditions, and
control means (7) for emitting an electric control signal to the balancing means (4), by means of which the change of the at least one parameter can be controlled,
wherein
the capacitor arrangement (21) without test material (9) is left temporally substantially unchanged,
an electric alternating signal is generated by the at least one alternating signal generator (3) and applied to the capacitor arrangement (21),
an electric output signal of the measuring circuit (2) is tapped,
at least one electric measuring quantity of the electric output signal tapped from the measuring circuit (2) is evaluated by the evaluation circuit (6),
at least one parameter of the electric alternating signal in the electric path between the at least one alternating signal generator (3) and the measuring circuit (2) is changed by the balancing means (4) in such a way that an output signal of the evaluation assumes a specific value, preferably zero, under defined constant conditions,
the change of the at least one parameter is controlled by the control means (7) by means of the electric control signal, and
the electric control signal is influenced by the output signal.

2. The method according to claim 1, wherein the influence on the electric control signal by the output signal occurs automatically in a closed feedback control loop.

3. The method according to one of the preceding claims, wherein the apparatus (1) comprises a feedback, by means of which an output signal of the measuring circuit (2) or of the evaluation circuit (6) acts upon the control means (7).

4. The method according to one of the preceding claims, wherein the balancing means (4) contain a plurality of electric resistors (421, 431, 433) which can be activated and deactivated individually or in groups.

5. The method according to one of the preceding claims, wherein the balancing means (4) contain a modulator (442) for an amplitude modulation of the electric alternating signal.

6. The method according to one of the preceding claims, wherein the balancing means (4) contain an amplifier (452, 462) with variable or programmable gain for amplifying the electric alternating signal.

7. The method according to one of the preceding claims, wherein the balancing means (4) contain a digital potentiometer or a rejustor (471, 481).

8. The method according to one of the preceding claims, wherein the balancing means (4) contain a variable-capacitance diode (492).

## Revendications

1. Procédé d'alignement de la symétrie d'un dispositif (1) pour l'examen capacitif d'un article textile allongé à contrôler (9) tel qu'un ruban de carde, une mèche, un fil ou un tissu au moyen d'une disposition de condensateurs (21), lequel dispositif (1) comprend
au moins un générateur de signaux alternatifs (3) pour appliquer un signal alternatif électrique à la disposition de condensateurs (21),
un circuit d'analyse (6) pour l'analyse d'au moins une grandeur de mesure électrique d'un signal capté sur un circuit de mesure (2) contenant la disposition de condensateurs (21),
des moyens d'alignement (4) qui se trouvent dans un chemin électrique entre l'au moins un générateur de signaux alternatifs (3) et le circuit de mesure (2) et au moyen desquels au moins un paramètre du signal alternatif électrique peut être modifié de telle manière qu'un signal de sortie du circuit d'analyse (6) prenne, dans des conditions définies et constantes, une certaine valeur, de préférence zéro, et
des moyens de commande (7) pour émettre un signal de commande électrique pour les moyens d'alignement (4), au moyen duquel la modification de l'au moins un paramètre peut être commandée,
dans lequel
la disposition de condensateurs (21) est laissée sensiblement inchangée dans le temps sans article à contrôler (9),
un signal alternatif électrique est généré par l'au moins un générateur de signaux alternatifs (3) et appliqué à la disposition de condensateurs (21),
un signal de sortie électrique du circuit de mesure (2) est capté,
au moins une grandeur de mesure électrique du signal de sortie électrique capté sur le circuit de mesure (2) est analysée par le circuit d'analyse (6),
au moins un paramètre du signal alternatif électrique est modifié par les moyens d'alignement (4) dans le chemin électrique entre le générateur de signaux alternatifs (3) et le circuit de mesure (2) de telle manière qu'un signal de sortie de l'analyse prenne, dans des conditions définies et constantes, une certaine valeur, de préférence zéro,
la modification de l'au moins un paramètre est commandée par les moyens de commande (7) avec le signal de commande électrique, et
le signal de commande électrique est influencé par le signal de sortie.

2. Procédé selon la revendication 1, dans lequel l'influence exercée sur le signal de commande électrique par le signal de sortie se produit automatiquement dans un circuit de régulation fermé.

3. Procédé selon l'une des revendications précédentes, dans lequel le dispositif (1) présente un couplage de rétroaction au moyen duquel un signal de sortie du circuit de mesure (2) ou du circuit d'analyse (6) agit sur les moyens de commande (7).

4. Procédé selon l'une des revendications précédentes, dans lequel les moyens d'alignement (4) contiennent plusieurs résistances électriques (421, 431, 433) qui peuvent être mises en circuit ou hors circuit séparément ou par groupes.

5. Procédé selon l'une des revendications précédentes, dans lequel les moyens d'alignement (4) comprennent un modulateur (442) pour une modulation d'amplitude du signal alternatif électrique.

6. Procédé selon l'une des revendications précédentes, dans lequel les moyens d'alignement (4) comprennent un amplificateur (452, 462) à amplification variable ou programmable pour amplifier le signal alternatif électrique.

7. Procédé selon l'une des revendications précédentes, dans lequel les moyens d'alignement (4) comprennent un potentiomètre numérique ou une résistance ré-ajustable (471,481).

8. Procédé selon l'une des revendications précédentes, dans lequel les moyens d'alignement (4) comprennent une diode capacitive (492).
